# FASCICULE DE BREVET EUROPEEN

(11) **EP 2 414 319 B1**
(45) Date de publication et mention de la délivrance du brevet: **01.07.2015**
(21) Numéro de dépôt: 10726144.8
(22) Date de dépôt: 26.03.2010
(51) Int. Cl.: C07C 229/26, C12P 13/00, C07C 227/18

(54) **METALLOPHORE DERIVE DE NICOTIANAMINE ET SES PROCEDES DE FABRICATION**
VON NICOTIANAMIN ABGELEITETES METALLOPHOR UND VERFAHREN ZU DESSEN HERSTELLUNG
NICOTIANAMINE-DERIVED METALLOPHORE AND PROCESSES FOR PRODUCING SAME

(30) Priorité: 31.03.2009 FR 0901574
(43) Date de publication de la demande: 08.02.2012
(73) Titulaire: Commissariat à l'Énergie Atomique et aux Énergies Alternatives, 75015 Paris (FR); Centre National de la Recherche Scientifique, 75016 Paris (FR)
(72) Inventeur: DREYFUS, Cyril, F-34920 Le Crès (FR); PIGNOL, David, F-04100 Manosque (FR); ARNOUX, Pascal, F-04110 Reillanne (FR); CAVELIER, Florine, F-34170 Castelnau le Lez (FR); LARROUY, Manuel, FR-06100 Nice (FR); MARTINEZ, Jean, F-34720 Caux (FR)
(74) Mandataire: Noel, Chantal Odile
(86) Numéro de dépôt international: PCT/FR2010/000261
(87) Numéro de publication internationale: WO 2010/112697

(56) Documents cités:
- US-A1- 2007 043 018
- DREYFUS, C., PIGNOL, D. & ARNOUX, P.: "Expression, purification, crystallization and preliminary X-ray analysis of an archaeal protein homologous to plant nicotianamine synthase" ACTA CRYSTALLOGRAPHICA SECTION F, vol. 64, 2008, pages 933-935, XP008115042 cité dans la demande

## Description

L'invention se rapporte à un dérivé de la nicotianamine et à un procédé de synthèse chimique ou enzymatique de celui-ci.

La nicotianamine (NA) est un acide aminé non-protéinogène, présent chez toutes les plantes supérieures. *In vitro*, la NA peut chélater de nombreux ions métalliques, (Fe, Mn, Zn, Ni, Cu), et *in vivo* elle joue un rôle important dans l'homéostasie de ces métaux chez les plantes.

En outre, il a été montré que la nicotianamine possédait des propriétés anti-hypertensives, par inhibition de l'enzyme de conversion de l'angiotensine I (ACE) (KINOSHITA et al., Biosci Biotechnol Biochem, 57, 1107-10, 1993; SHIMIZU et al., J Nutr Sci Vitaminol (Tokyo), 45, 375-83, 1999; WADA et al., Biosci Biotechnol Biochem, 70, 1408-15, 2006).

US2007/0043018 divulgue la nicotianamine et la 2"-hydroxynicotianamine comme inhibiteurs de l'ACE.

Chez les plantes supérieures, la biosynthèse de la nicotianamine est catalysée par la nicotianamine synthase (NAS; EC 2.5.1.43) et résulte de la trimérisation de trois molécules de S-adénosyl méthionine (SAM). Des recherches sur les bases de données de séquences ont permis de détecter des homologues putatifs de NAS chez d'autres organismes que les plantes supérieures, notamment chez des champignons et des archaes (TRAMPCZYNSKA et al., FEBS Lett, 580, 3173-8, 2006; HERBIK et al., Eur J Biochem, 265, 231-9, 1999). La présence d'une NAS fonctionnelle a en outre été démontrée chez le champignon filamenteux *Neurospora crassa* (TRAMPCZYNSKA et al., FEBS Lett, 580, 3173-8, 2006). Récemment, l'équipe des inventeurs (DREYFUS et al., Acta Cryst., F64, 933-5, 2008) a exprimé chez *E. coli* une enzyme de l'archae *Methanothermobacter thermoautotrophicus*, (MTH675 ; GenBank NP_275817) homologue des NAS eucaryotes. Cette enzyme sera dénommée ci-après MtNAS.

En poursuivant leurs travaux sur la caractérisation de cette enzyme de *Methanothermobacter thermoautotrophicus,* les inventeurs ont mis en évidence une nouvelle molécule synthétisée par celle-ci.

Cette molécule, dénommée ci-après thermonicotianamine (thNA), comporte un groupement carboxylique supplémentaire par rapport à la nicotianamine.

Ce dérivé de la nicotianamine a la formule suivante :

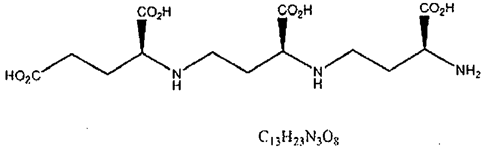

Ce dérivé peut avantageusement être synthétisé par un procédé chimique qui comprend les étapes suivantes :
a) la protection de la fonction amine de l'α-tert-butyl ester de l'acide L-aspartique,
b) la réduction de la fonction carboxylique du composé obtenu à l'étape a),
c) l'halogénation de la fonction alcool du composé obtenu à l'étape b), de préférence avec l'iode,
d) la protection-activation de la fonction amine du di-tert- butyl ester de l'acide L-glutamique,
e) l'alkylation du composé obtenu à l'étape d), avec le composé obtenu à l'étape c),
f) la déprotection suivie de la protection-activation de la fonction amine du composé obtenu à l'étape e),
g) l'alkylation du composé obtenu à l'étape f), avec le composé obtenu à l'étape c), et
h) la déprotection totale du composé obtenu à l'étape g).

La thermonicotianamine peut également être synthétisée par voie enzymatique, par incubation des substrats SAM et acide glutamique (GLU) en présence de l'enzyme MtNAS de *méthanothermobacter*, ou l'un de ses orthologues chez une autre archae, purifiée comme décrit par DREYFUS et al. (2008, précité).

On peut également le synthétiser à l'aide de cultures de *thermoautotrophicus* ou d'une autre archae exprimant un orthologue de MtNAS, ou d'une cellule-hôte (par exemple *E.coli*) transformée par un polynucléotide codant pour MtNAS ou l'un de ses orthologues, et exprimant ladite enzyme.

Le dérivé de la nicotianamine selon l'invention peut avantageusement être utilisé en remplacement ou en addition à la nicotianamine elle-même dans toutes les applications de la nicotianamine.

Le procédé chimique de synthèse du dérivé de la nicotianamine de l'invention se déroule selon le schéma réactionnel ci-après :

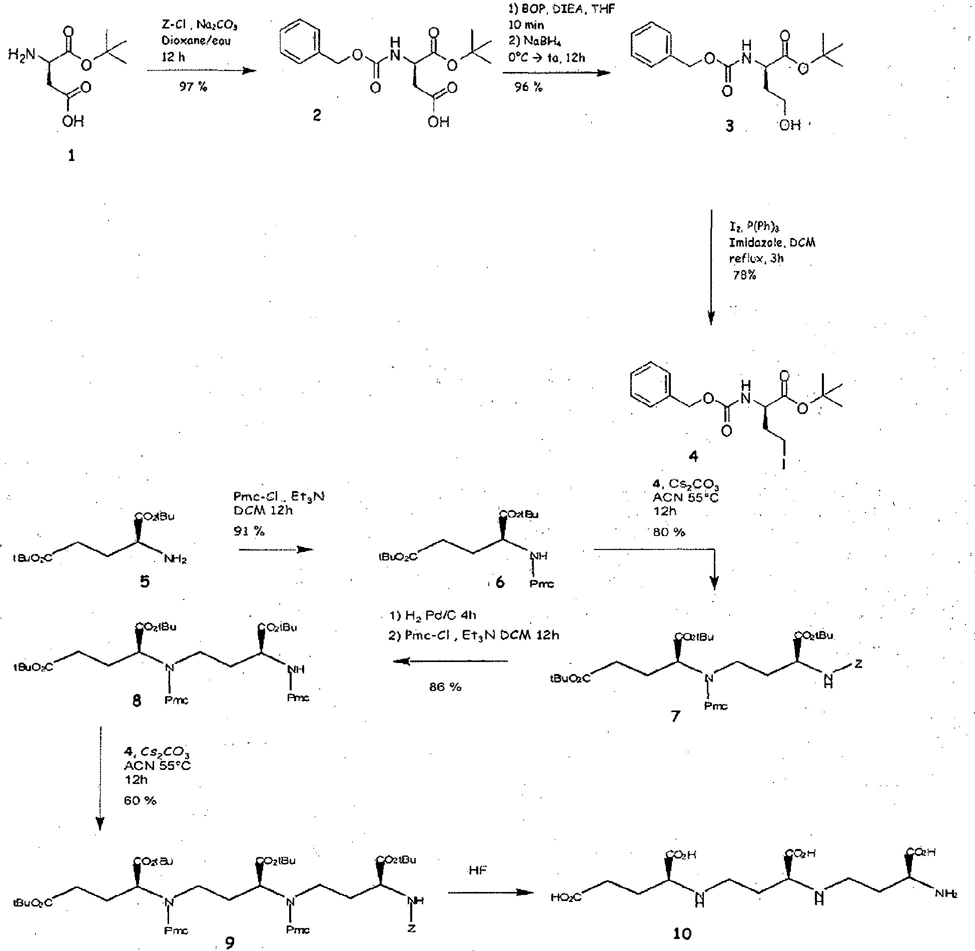

Plus précisément, le dérivé de la nicotianamine selon l'invention est fabriqué en partant de l'α-tert-butyl ester de l'acide L-aspartique qui est un produit commercialement disponible, d'une part, et du di-tert-butyl ester de l'acide L-glutamique qui est également un produit commercialement disponible, d'autre part.

On part donc de l'α-tert-butyl ester de l'acide L-aspartique qui est le composé nommé 1 de formule suivante :

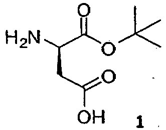

On procède à la protection de l'amine de ce composé de la façon suivante :

A une solution d'aspartate de α-*tert*-butyle (10 g, 53 mmol) dans un mélange eau/dioxane (2/1) (170mL) sont ajoutés du bicarbonate de sodium (13 g, 159 mmol) et sur une durée de 2 heures, une solution de chloroformiate de benzoyle (11,8 mL, 69 mmol) dans du dioxane (70 mL). L'agitation est maintenue pour la nuit à température ambiante. Le milieu réactionnel est lavé avec de l'acétate d'éthyle (3 x 200 mL), puis acidifié à 0°C par une solution d'acide chlorhydrique 6N jusqu'à pH = 2. La phase aqueuse est extraite avec de l'acétate d'éthyle (3 x 200 mL). Les phases organiques réunies sont lavées avec de la saumure (200 mL) puis séchées sur sulfate de magnésium anhydre et concentrées pour obtenir le composé **2** sous forme d'une huile incolore (16,7 g, 97 %).

On obtient le composé 2 de formule suivante : 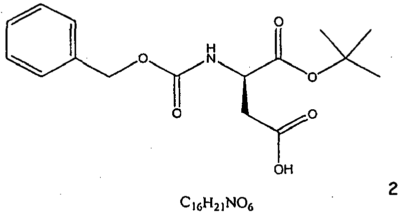
Rf : (CHCl₃/MeOH/AcOH 37%) = 0,68
HPLC : 1,56 min
MS : [M+H]⁺, 324,1 ; [M+H - tBu]⁺, 268,1
RMN ¹H (CDCl₃, 300 MHz) : 1,45 (s, 9H, 1 tBu) ; 2,95 (dd, 2H, J = 4,5 et 14 Hz, CH_{2β}) ; 4,55 (q, 1H, J = 4,3Hz, CH_{α}) ; 5,10 (s, 2H, CH₂Ph) ; 5,8 (d, 1H, J = 8 Hz, NH) ; 7,35 (m, 5H, arom) ; 10,7 (sl, 1H, COOH)
RMN ¹³C (CDCl₃, 75 MHz) : 27,79 ; 36,73 ; 50,75 ; 67,12 ; 82,83 ; 128,11 ; 128,20 ; 128,53 ; 136,12 ; 156,06 ; 169,46 ; 176,25

Puis on procède à la réduction de la fonction carboxylique du composé **2** de la façon suivante :

A une suspension de **2** (15 g, 46,4 mmol) et de BOP (27 g, 60,4 mmol) dans du THF anhydre est ajouté de la diisopropyléthylamine (10,5 mL, 60,4 mmol). Le milieu réactionnel est laissé sous vive agitation pendant 10 minutes jusqu'à ce qu'il se colore en jaune puis est refroidi à 0°C pendant 15 minutes. Ensuite est ajouté au milieu réactionnel NaBH₄ (2,3g, 60,4 mmol) par petites portions. Le mélange réactionnel est laissé sous vive agitation une nuit. Le solvant est évaporé et le résidu repris dans l'acétate d'éthyle (250 mL). La solution est lavée successivement avec une solution d'acide citrique à 10 % (3 x 100 mL), une solution saturée en hydrogénocarbonate de sodium (3 x 100 mL) puis de l'eau distillée (2 x 100 mL). La phase organique est séchée sur sulfate de magnésium anhydre puis concentrée pour donner une huile incolore. Le composé **3** est purifiée par chromatographie sur gel de silice (éther de pétrole/éther : 3/7) pour donner une huile incolore (13,95 g, 96%).

On obtient le composé **3** de formule suivante : 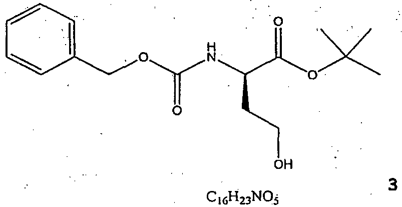
Rf : (éther de pétrole/éther : 3/7) = 0,42
HPLC : 1,45 min
MS : [M+H]⁺, 310,2 ; [M+H - tBu]⁺, 254,1
RMN ¹H (CDCl₃, 300 MHz) : 1,43 (s, 9H, 1 tBu) ; 2,09 (dd, 2H, J = 4,6 et 15,2 Hz, CH_{2β}) ; 3,48 (s, 1H, OH) ; 3,8 (m, 2H, CH_{2γ}) ; 4,45 (m, 1H, CH_{α}) ; 5,10 (s, 2H, CH₂Ph) ; 5,87 (d, 1H, J = 8 Hz, NH) ; 7,35 (m, 5H, arom)
RMN ¹³C (CDCl₃, 75 MHz) : 27,9 ; 35,45 ; 51,95 ; 58,4 ; 67,01 ; 82,44 ; 128,07 ; 128,13 ; 128,13 ; 128,31 ; 129,01 ; 136,1 ; 156,79 ; 169,46, 171,68.

Puis on procède à l'halogénation de l'alcool du composé **3** de la façon suivante :

A une solution du composé **3** (5,8 g, 18,8 mmol) dans du dichlorométhane anhydre (260 mL) sous argon, sont ajoutés de l'iode (14,3g, 56,4 mmol), de la triphénylphosphine (14,8 g, 56,4 mmol) et après 10 minutes, de l'imidazole (9,6 g, 141 mmol). Le milieu réactionnel est porté à reflux et est laissé sous vive agitation pendant 3 heures. Le mélange réactionnel, une fois revenu à température ambiante est lavé successivement avec une solution molaire d'hydrogénosulfate de potassium (3 x 150 mL), une solution saturée en hydrogénocarbonate de sodium (3 x 150 mL) une solution à 5 % de thiosulfate de sodium (3 x 150 mL) et avec de la saumure (150 mL). La phase organique est séchée sur sulfate de magnésium anhydre puis concentrée pour donner une pâte jaune. Le produit **4** est obtenu après chromatographie sur gel de silice (éther de pétrole/acétate d'éthyle : 10/0 à 8/2) sous forme d'une huile jaune (6,2 g 78 %).

On obtient le composé **4** de formule suivante : 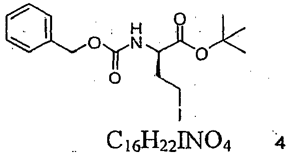
Rf : (éther de pétrole/acétate d'éthyle : 8/2) = 0,65
HPLC :
MS : [M+Na]⁺, 442,0 ; [M+H]⁺, 420,1 ; [M+H - tBu]⁺, 364,2
RMN ¹H (CDCl₃, 300 MHz) : 1,45 (s, 9H, 1 tBu) ; 2,15-2,45 (2m, 2H, CH_{2β}) ; 3,15 (m, 2H, CH_{2γ}) ; 4,35 (m, 1H, CH_{α}) ; 5,10 (s, 2H, CH₂Ph) ; 5,87 (d, 1H, J = 8 Hz, NH) ; 7,35 (m, 5H, arom)
RMN ¹³C (CDCl₃, 75 MHz) : 16,1 ; 28,42 ; 38,45 ; 54,24 ; 56,15 ; 67,89 ; 83,59 ; 128,95 ; 129,03 ; 129,22 ; 129,34 ; 129,38 ; 136,93 ; 156,69 ; 171,03.

Séparément, on prend le di-tert-butyl ester de l'acide L-glutamique : composé **5** et on procède à la protection-activation de l'amine de ce composé **5** de formule suivante :

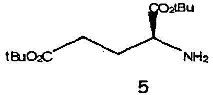

A une solution du chlorhydrate du glutamate de *tert*-butyle (0,62 g, 2,10 mmol) dans du dichlorométhane anhydre (20 mL) est ajouté de la triéthylamine (0,29 mL, 2,10 mmol). La solution est filtrée puis y est ajouté de la triéthylamine (0,60 mL, 4,41 mmol) et du chlorure de 2,2,5,6,7-pentaméthylchromane sulfonyle (0,7 g, 3,30 mmol) dissous dans du dichlorométhane anhydre (4 mL) sur une durée de 2 h avec un pousse seringue. Le milieu réactionnel est laissé sous vive agitation une nuit. Le milieu réactionnel est concentré sous vide puis repris dans l'acétate d'éthyle (20 mL). La phase organique est successivement lavée avec une solution à 10 % d'acide citrique (3 x 20 mL), avec une solution saturée en hydrogénocarbonate de sodium (3 x 20 mL), avec de l'eau distillée (3 x 20 mL) puis séchée sur sulfate de magnésium anhydre et concentrée sous vide. Le sulfonamide **6** est obtenu après chromatographie sur gel de silice (éther de pétrole/éther 8/2) sous forme d'une huile incolore (1 g, 91 %).

On obtient le composé **6** suivant : 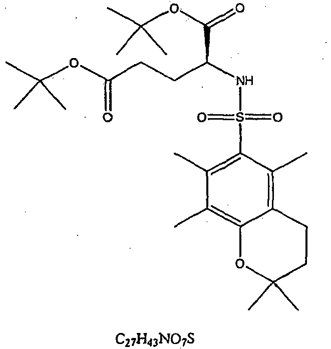
Rf : (éther de pétrole/éther 6/4) = 0,3
HPLC : 2,41 min
MS : [M+H]⁺, 526,3 ; [M+H - tBu]⁺, 470,2 ; [M+H- 2 tBu]⁺, 414,2
RMN ¹H (CDCl₃, 300 MHz) : 1,14-1,24 (s, 9H, 1 tBu) ; 1,22-1,24 (2s, 6H, 2 CH₃) ; 1,36 (s, 9H, 1 tBu) ; 1,70-1,99 (m, 4H, CH₂ *méta*, CH_{2β}) ; 2,03 (s, 3H, CH₃) ; 2,27-2,33 (m, 2H, CH_{2γ}) ; 2,47-2,49 (2s, 6H, 2 CH₃ *ortho*) ; 2,54-2,59 (t, 2H, J = 6,8 et 6,8 Hz, CH₂) ; 3,66-3,67 (m, 1H, CH_{α}) ; 5,20-5,23 (d, 1 H, J = 9,4 Hz, NH) RMN ¹³C (CDCl₃, 75 MHz) : 11,42 ; 12,17 ; 14,11 ; 17,30 ; 18,25 ; 19,42 ; 20,42 ; 21,50 ; 22,60 ; 26,52 ; 26,81 ; 27,60 ; 28,05 ; 28,60 ; 29,04 ; 29,68 ; 30,97 ; 32,67 ; 55,09 ; 73,91 ; 76,61 ; 80,53 ; 82,29 ; 118,32 ; 124,60 ; 128,43 ; 136,42 ; 136,60 ; 154,65 ; 171,07 ; 171,97.

Puis on procède à l'alkylation du composé **6** avec le composé **4** de la façon suivante :

A une solution du sulfonamide **6** (0,81 g, 1,54 mmol) dans l'acétonitrile anhydre (20 mL) est additionné du carbonate de césium (0,75 g, 2,31 mmol). Le milieu réactionnel est mis sous vive agitation pendant 30 minutes, puis chauffé à 55°C. Ensuite est additionné très lentement au pousse seringue le composé iodé **4** (0,64 g, 1,54 mmol) en solution dans de l'acétonitrile anhydre (5 mL). Le milieu réactionnel est laissé sous vive agitation à 55°C pendant 1 nuit. Le milieu réactionnel est évaporé sous vide puis repris dans l'acétate d'éthyle (20 mL). La phase organique est successivement lavée avec de la saumure (3 x 20 mL) et avec de l'eau distillée (3 x 20 mL), puis séchée sur sulfate de magnésium anhydre et concentrée sous vide. Le sulfonamide **7** est obtenu après chromatographie sur gel de silice (éther de pétrole/éther 7/3) puis dichlorométhane/éther 99/1 à 98/2) sous forme d'une huile incolore (1g, 80 %).

On obtient le composé **7** de formule suivante : 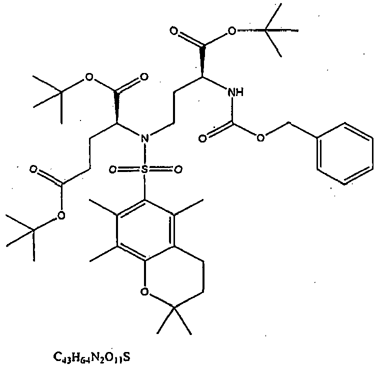
Rf : (éther de pétrole/éther 6/4) = 0,57
HLPC : 2,72 min
MS : [M+Na]⁺, 839,6 ; [M+H]⁺, 817,6 ; [M+H - tBu]⁺ ; 761,5 ; [M+H- 2 tBu]⁺, 705,5 ; [M+H- 3 tBu]⁺, 649,5
RMN ¹H (CDCl3, 300 MHz) : 1,17-1,36 (5s, 33H, 3 tBu, 2 CH₃) ; 1,70-2,00 (m, 6H, CH₂, CH_{2β}, CH_{2β'}) ; 2,03 (s, 3H, CH₃ *méta*) ; 2,17-2,20 (m, 2H, CH_{2γ}) ; 2,41-2,42 (2s, 6H, 2 CH₃ *ortho*) ; 2,52-2,56 (t, 2H, J = 6,8 et 6,3 Hz, CH₂) ; 3,27-3,52 (m, 2H, CH_{2γ'}) ; 4,01-4,06 (m, 1H, CH_{α}) ; 4,10-4,13 (t, 1H, CH_{α'}) 5,02-5,03 (s, 2H, CH₂Ph) ; 5,19-5,25 (1H, J = 6 Hz, NH) ; 7,24-7,29 (m, 5H, arom)
RMN ¹³C (CDCl₃, 75 MHz) : 11,06 ; 16,04 ; 17,02 ; 18,21 ; 20,31 ; 21,39 ; 24,41 ; 25,40 ; 25,54 ; 26,55 ; 26,63 ; 26,80 ; 27,82 ; 28,46 ; 30,62 ; 31,34 ; 31,42 ; 39,42 ; 51,63 ; 56,97 ; 65,60 ; 72,78 ; 75,40 ; 79,31 ; 80,73 ; 81,00 ; 117,24 ; 123,48 ; 126,42 ; 126,82 ; 127,23 ; 135,16 ; 136,47 ; 136,66 ; 153,79 ; 154,78 ; 169,06 ; 169,60 ; 170,24.

Puis on procède à la déprotection du composé **7** suivie par la protection-activation de la fonction amine de ce composé déprotégé de la façon suivante :

Au composé 7 (0,48 g, 0,59 mmol) dissous dans du THF anhydre (7mL) est ajouté du palladium sur charbon (150 mg, 30 % en masse). L'air du montage est éliminé et remplacé par de l'argon par 3 cycles de vide-argon, puis par de l'hydrogène par 3 cycles de vide-hydrogène. Le milieu réactionnel est laissé sous vive agitation pendant 6 heures puis filtré sur célite et concentré. Le mélange réactionnel est dissous dans du dichlorométhane anhydre (5 mL) puis, sont ajoutés de la triéthylamine (0.20 mL, 1,18 mmol) puis sont additionnés goutte à goutte le chlorure de 2,2,5,6,7-pentaméthylchromane sulfonyle (231 mg, 0,768 mmol) dissous dans du dichlorométhane anhydre (4 mL) sur une durée de 2 h. Le milieu réactionnel est laissé sous vive agitation une nuit. Le milieu réactionnel est concentré sous vide puis repris dans l'éther (10 mL). La phase organique est successivement lavée avec une solution à 10 % d'acide citrique (3 x 10 mL), avec une solution saturée en hydrogénocarbonate de sodium, (3 x 10 mL) et avec de l'eau distillée (3 x 10 mL) puis séchée sur sulfate de magnésium anhydre et concentrée sous vide. Le disulfonamide **8** est obtenu après chromatographie sur gel de silice (éther de pétrole/éther 6/4) sous forme d'une huile incolore (480 mg, 86 %).

On obtient le composé 8 suivant : 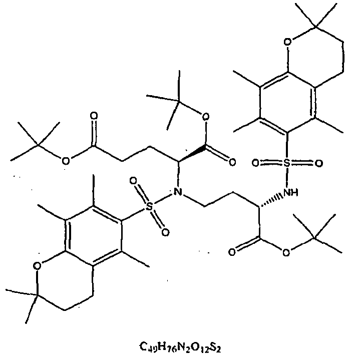
Rf : (éther de pétrole/éther 6/4) = 0,27
MS : [M+Na]⁺, 971,6; [M+H]⁺, 946,6 ; [M+H - tBu]⁺, 893,5 ; [M+H- 2 tBu]⁺; 837,4 ; [M+H- 3 tBu]⁺, 781,3
RMN ¹H (CDCl₃, 300 MHz) : 1,13-1,33 (5s, 39H, 3 tBu, 6 CH₃) ; 1,71-1,82 (m, 4H, CH₂) ; 1,94-2,03 (m, 10H, 2 CH₃ *méta*, CH_{2β}, CH_{2β'}) ; 2,14-2,17 (t, 2H, CH_{2γ}) ; 2,41-2,42 (2s, 6H, 2 CH₃ *ortho*); 2,46-2,48 (2s, 6H, 2 CH₃ *ortho*) ; 2,55-2,59 (t, 4H, J = 8,6 et 6,5 Hz, CH₂) ; 3,40-3,46 (t, 2H, J = 7,9 et 8,7 Hz, CH_{2βγ'}) ; 3,52-3,61 (m, 1H, CHα') 3,99-4,06 (m, 1H, CH_{α}); 5,19-5,22 (1H, J = 8,3Hz, NH)
RMN ¹³C (CDCl₃, 75 MHz) : 12,30 ; 13,05 ; 13,15 ; 15,19 ; 18,19 ; 19,12 ; 19,15 ; 19,63 ; 20,30 ; 22,35 ; 22,44 ; 23,48 ; 26,37 ; 27,49 ; 27,51 ; 27,65 ; 28,42 ; 28,88 ; 29,91 ; 30,56 ; 32,55,; 33,54 ; 34,08 ; 41,40 ; 55,11 ; 59,08 ; 74,84 ; 81,32 ; 82,67 ; 83,38 ; 103,68 ; 119,20 ; 119,38 ; 125,46 ; 125,51 ; 128,47 ; 129,24 ; 137,36 ; 137,56 ; 138,68; 138,76 ; 155,53 ; 155,84 ; 171,13; 171,40; 172,31,

On procède à une nouvelle alkylation du composé **8** avec le composé **4** de la façon suivante:

A une solution du sulfonamide **8** (121 mg, 0,128 mmol) dans de l'acétonitrile anhydre (600 µL) sous atmosphère inerte est additionné du carbonate de césium (75 mg, 0,231 mmol). Le milieu réactionnel est mis sous vive agitation pendant 30 minutes, puis est chauffé à 55°C. Ensuite est additionné très lentement au pousse seringue le composé iodé **4** (107 mg, 0,256 mmol) en solution dans de l'acétonitrile anhydre (600 µL). Le milieu réactionnel est laissé sous vive agitation à 55°C pendant 1 nuit. Le milieu réactionnel est concentré sous vide puis repris dans du cyclohexane (5 mL). La phase organique est lavée avec de l'eau distillée (3 x 5 mL), puis séchée sur sulfate de magnésium anhydre et concentrée sous vide. Le disulfonamide **9** est obtenu après chromatographie sur gel de silice (dichlorométhane/éther 97/3) sous forme d'une huile incolore (99 mg, 60 %).

On obtient le composé **9** suivant : 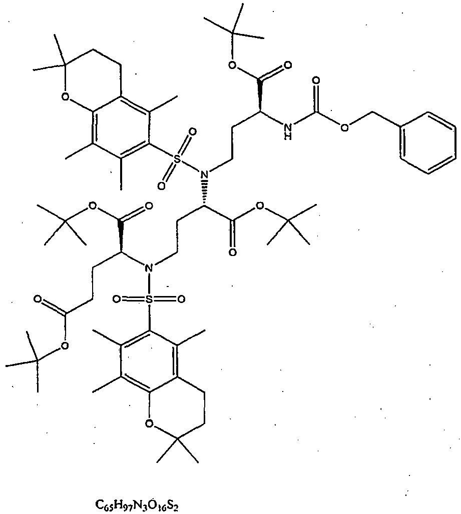
Rf : (dichlorométhane/éther 96/4) = 0,26
MS : [M+Na]⁺, 1262,5 ; [M+H]⁺, 1240,5 ; [M+H - tBu]⁺, 1184,4 ; [M+H- 2 tBu]⁺, 1128,4 ; [M+H- 3 tBu]⁺,1072,4 ; [M+H- 4 tBu]⁺,1016,3
RMN ¹H (CDCl₃, 300 MHz) : 1,22-1,37 (5s, 48H, 3 tBu, 6 CH₃ ) ; 1,70-1,75 (t, 4H, J = 6,4 et 6,7 Hz, CH₂) ; 1,78-2,04 (m, 12H, 2 CH₃ *méta*, CH_{2β}, CH_{2β'}, CH_{2β"}); 2,17-2,20 (m, 2H, CH_{2γ}) ; 2,40-2,44 (2s, 6H, 2 CH₃ *ortho*) ; 2,46-2,48 (2s, 6H, 2 CH₃ *ortho*) ; 2,55-2,58 (t, 4H, J = 6,5 et 6,6 Hz, CH₂) ; 3,33-3,44 (m, 4H, J = CH_{2γ'}, CH_{2γ"}); 3,95-4,03 (2m, 3H, CH_{α}, CH_{α'}, CH_{α"}) ; 5,00-5,02 (d, 2H, J = 6,8Hz, CH₂Ph) ; 7,22-7,26 (m, 5H, arom)
RMN ³C (CDCl₃, 75 MHz) : 12,30 ; 13,05 ; 13,15 ; 15,19 ; 18,19 ; 19,12 ; 19,15 ; 19,63 ; 20,30 ; 22,35 ; 22,44 ; 23,48 ; 26,37 ; 27,49 ; 27,51 ; 27,65 ; 28,42 ; 28,88 ; 29,91 ; 30,56 ; 32,55 ; 33,54 ; 34,08 ; 41,40 ; 55,11 ; 59,08 ; 74,84 ; 81,32 ; 82,67 ; 83,38 ; 103,68 ; 119,20 ; 119,38 ; 125,46 ; 125,51 ; 128,47 ; 129,24 ; 137,36 ; 137,56 ; 138,68 ; 138,76 ; 155,53 ; 155,84 ; 171,13 ; 171,40 ; 172,31,

Enfin, on procède à la déprotection du composé **9** de la façon suivante:

Dans un réacteur en téflon contenant le composé **9** (79 mg, 0,064 mmol) est additionné l'anisole (200 µL, 10% v/v) puis le fluorure d'hydrogène (2 mL, en excès) à l'aide d'un banc en téflon. Le milieu réactionnel est laissé sous vive agitation à 0°C pendant 9 heures. L'excès de fluorure d'hydrogène est neutralisé sur potasse. Le résidu est trituré à l'éther (3 x 3 mL) puis repris dans une solution à 0.1 M d'acide chlorhydrique. L'eau est éliminée par lyophilisation et le composé **10** est obtenu pur sous la forme d'un solide blanc.

On obtient le composé **10** qui est le dérivé de la nicotianamine selon l'invention de formule suivante : 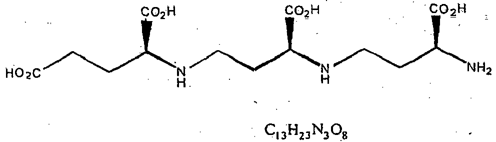
MS:[M+H]⁺, 350,1 ; [M+H-18]⁺, 332,1

## Revendications

1. Dérivé de la nicotianamine **caractérisé en ce qu'**il a la formule suivante :

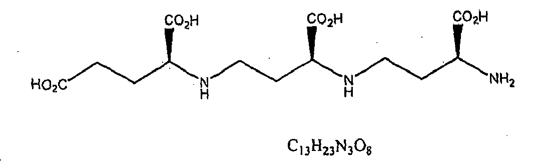

2. Procédé de synthèse chimique du dérivé de la nicotianamine selon la revendication 1 **caractérisé en ce qu'**il comprend les étapes suivantes :
a) la protection de la fonction amine de l'α-tert-butyl ester de l'acide L-aspartique,
b) la réduction de la fonction carboxylique du composé obtenu à l'étape a),
c) l'halogénation de la fonction alcool du composé obtenu à l'étape b), de préférence avec l'iode,
d) la protection-activation de la fonction amine du di-tert- butyl ester de l'acide L-glutamique,
e) l'alkylation du composé obtenu à l'étape d), avec le composé obtenu à l'étape c),
f) la déprotection suivie de la protection-activation de la fonction amine du composé obtenu à l'étape e),
g) l'alkylation du composé obtenu à l'étape f), avec le composé obtenu à l'étape c), et
h) la déprotection totale du composé obtenu à l'étape g).

3. Procédé de synthèse enzymatique du dérivé de la nicotianamine selon la revendication 1 par incubation des substrats S-Adenosylméthionine et acide glutamique en présence de l'enzyme MtNAS de *Méthanothermobacter*, ou l'un de ses orthologues chez une autre archae.

## Patentansprüche

1. Nikotinaminderivat, **gekennzeichnet durch** folgende Formel:

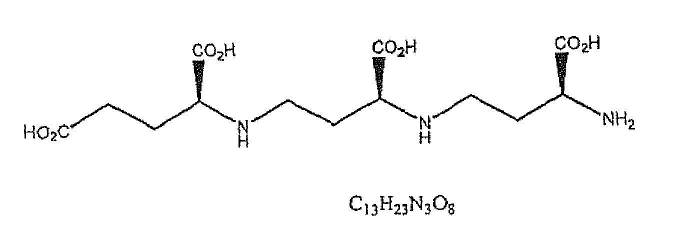

2. Chemisches Syntheseverfahren des Nikotinaminderivates gemäß Anspruch 1, **dadurch gekennzeichnet, dass** es folgende Stufen umfasst:
a) Schutz der Aminfunktion des α-tert-Butylesters der Asparaginsäure,
b) Reduktion der Carboxylfunktion der in Stufe a) erhaltenen Verbindung,
c) Halogenierung der Alkoholfunktion der in Stufe b) enthaltenen Verbindung, vorzugsweise mit Jod,
d) Schutz-Aktivierung der Aminfunktion des Di-tert-Butylesters der Glutaminsäure,
e) Alkylierung der in Stufe d) erhaltenen Verbindung mit der in Stufe c) erhaltenen Verbindung,
f) Entfernung des Schutzes gefolgt von Schutz-Aktivierung der Aminfunktion der Verbindung, welche in Stufe e) erhalten wurde,
g) Alkylierung der in Stufe f) erhaltenen Verbindung mit der in Stufe c) erhaltenen Verbindung, und
h) Entfernen des gesamten Schutzes der in Stufe g) erhaltenen Verbindung.

3. Enzymatisches Syntheseverfahren des Nikotinaminderivates gemäß Anspruch 1 durch Inkubation der Substrate S-Adenosylmethionin und Glutaminsäure in Gegenwart des Enzyms MtNAS aus *Methanothermobacter* oder einem seiner Orthologen aus einem anderen Archaebakterium.

## Claims

1. Nicotianamine derivative, **characterised in that** it has the following formula:

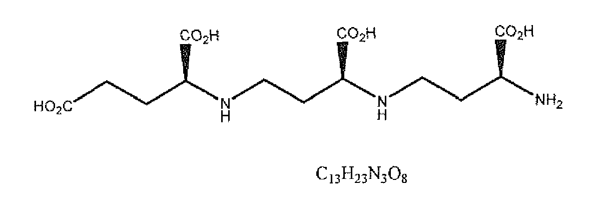

2. Process for the chemical synthesis of the nicotianamine derivative according to claim 1, **characterised in that** it comprises the following steps:
a) protection of the amine function of the α-tert-butyl ester of L-aspartic acid,
b) reduction of the carboxylic function of the compound obtained in step a),
c) halogenation of the alcohol function of the compound obtained in step b), preferably with iodine,
d) protection-activation of the amine function of the di-tert-butyl ester of L-glutamic acid,
e) alkylation of the compound obtained in step d) with the compound obtained in step c),
f) deprotection of the compound obtained in step e) followed by protection-activation of the amine function thereof,
g) alkylation of the compound obtained in step f) with the compound obtained in step c), and
h) full deprotection of the compound obtained in step g).

3. Process for the enzymatic synthesis of the nicotianamine derivative according to claim 1 by incubation of the substrates S-adenosylmethionine and glutamic acid in the presence of the MtNAS enzyme of *Methanothermobacter,* or an orthologue thereof in a different archaea.
